# EUROPEAN PATENT APPLICATION

(11) **EP 4 399 980 A1**
(43) Date of publication of application: **17.07.2024**
(21) Application number: 22864748.3
(22) Date of filing: 05.09.2022
(51) Int. Cl.: A23L 33/13, A61K 31/706, A61P 9/00, A61P 9/04, A61P 9/06, A61P 9/10

(54) **HEART DISEASE AGENT AND METHOD FOR IMPROVING HEART DISEASE**

(30) Priority: 06.09.2021 JP 2021144496
(71) Applicant: Mirailab Bioscience Inc., Tokyo 104-0061 (JP)
(72) Inventor: TANAKA, Megumi, Chigasaki-shi, Kanagawa 253-0054 (JP); TANAKA, Tsunemaru, Chigasaki-shi, Kanagawa 253-0054 (JP)
(74) Representative: IPAZ
(86) International application number: PCT/JP2022/033223
(87) International publication number: WO 2023/033166

(57) **Abstract**

[PROBLEM] To provide a medicine for heart disease that is safe for use over a long period of time and useful for effectively improving heart diseases.

[SOLUTION] The present invention is a medicine for heart disease containing nicotinamide mononucleotide as an active ingredient.

## Description

### Technical Field

The present invention is a medicine for heart disease containing nicotinamide mononucleotide as an active ingredient and a method for improving heart disease.

### Background Art

The heart is an organ that takes pumping action to send blood to peripheral blood vessels, which is the most major organ in the circulatory system. The above-described pumping action is performed by the atriums and ventricles of the heart alternately repeating contraction and dilation with regularity. The heart has a special heart muscle called a stimulus conduction system that generates a pulsed electrical signal and transmits the electrical signal to individual myocardial fibers. That is, the heart is made of a special muscle called the heart muscle and gets excited by an electric current flowing through the heart muscle to move. The starting point of the stimulus conduction system is the sinoatrial node where an electrical signal with about 70 pulses per minute is correctly generated and acts as a pacemaker for cardiac contraction. Afterwards, the electrical signal from the sinoatrial node is transmitted to the atrial muscle, causing contraction of the atriums. Next, electrical stimulation is transmitted to the atrioventricular node, and a signal from the atrioventricular node spreads to the bundle of His and Purkinje fibers to transmit the electrical stimulation throughout the heart muscle. Thus, the contraction and dilation of the heart muscle are maintained in a constant rhythm by the electrical signal.

An arrhythmia is a condition in which the rhythm of the heart's electrical signal flow is abnormal due to disorders generated in this stimulus conduction system. Arrhythmias are broadly divided into tachycardia, which causes a pulse to get faster, bradycardia, which causes a pulse to get slower, and extrasystoles where a pulse comes from a place different from normal ahead of schedule. Since an abnormal pulse that is generated suddenly not only induces palpitations, shortness of breath, chest discomfort, and dizziness but also threatens life in some cases, appropriate responses andtreatments are necessary. The causes of arrhythmias include, for example, aging, physical constitution, stress, lack of sleep, fatigue, alcohol drinking, smoking, and factors related to autonomic nerves. As another cause, when the heart is damaged by heart disease, such as myocardial infarction and angina pectoris, disorders also occur in the stimulus conduction system, therefore easily causing arrhythmias. Conversely, arrhythmias, such as atrial fibrillation and ventricular fibrillation, cause heart diseases, such as cardiomyopathy, valvular disease, and ischemic heart disease. Furthermore, arrhythmias can be caused by side effects of medicines taken for the treatment of heart diseases and the like.

The classification and type of anti-arrhythmic drugs include the Sicilian Gambit classification, which takes into account the mechanism of development of arrhythmias and the effect of each anti-arrhythmic drug on molecular targets, such as ion channels and receptors, and a framework for the classification of anti-arrhythmic drugs is proposed to select an appropriate anti-arrhythmic drug. However, since the anti-arrhythmic drugs can cause serious side effects and lead to sudden death if indications or dosage are wrong, they are classified as high-risk drugs that require particular safety management. Therefore, it is necessary to sufficiently confirm that the selection, dose regimen, and dosage of the anti-arrhythmic drug are appropriate.

The anti-arrhythmic drugs conventionally used include Na channel blockers (classes Ia group, Ib group, and Ic group), β-blockers (class II group), αβ-blockers, potassium channel blockers (class III group), Ca antagonists (class IV group), digitalis preparations, and the like. In recent years, new anti-arrhythmic drugs have been developed one after another. As an example, an anti-arrhythmic agent including 9-β-D-arabinofuranosylhypoxanthine as a metabolite of vidarabine used clinically as an antiviral drug, and a pharmaceutically acceptable salt thereof or a solvate of the same has been proposed by focusing on the signaling system downstream of the β-adrenergic receptor (Patent Document 1) .

### Citation List

### Patent Literature

Patent Literature 1: WO 2019-131308

### Summary of Invention

### Technical Problem

The anti-arrhythmic drugs are known to cause new arrhythmias on their own (proarrhythmic effects). Especially for the elderly, severe tachycardiac and bradycardiac proarrhythmic effects of medicines easily emerge due to aging-related changes in renal function and heart muscle stimulus conduction system. In addition, it has been reported that depending on the type of the anti-arrhythmic drugs, they cause various extracardiac side effects, such as urinary retention, optic nerve neuropathy due to increased ocular pressure, general malaise, sleep disorder, depression tendencies, and hypoglycemia. Therefore, it is necessary to constantly monitor electrocardiograms carefully and perform blood biochemistry tests to attempt to detect and avoid the side effects of the anti-arrhythmic drugs at an early stage. As described above, the anti-arrhythmic drugs can cause severe side effects and lead to sudden death if indications or dosage are wrong. With such a situation as background, there is a need for a highly safe medicine that is superior in improving arrhythmias and has few side effects.

An object of the present invention is to provide a medicine for heart disease that is safe for use over a long period of time and useful for preventing and treating heart diseases related to arrhythmias.

### Solution to Problem

As a result of serious study for solving the above-described problems, the inventor discovered that nicotinamide mononucleotide, which is an intermediate metabolite related to biosynthesis of coenzyme nicotinamide adenine dinucleotide (NAD), has a beneficial prevention and improving effect on heart diseases, and completed the present invention.

The present invention is as follows.
[1] A medicine for heart disease that contains nicotinamide mononucleotide as an active ingredient.
[2] The medicine for heart disease according to [1] where the heart disease is a condition presenting with an arrhythmia, a disease causing an arrhythmia, or a disease caused by an arrhythmia.
[3] The medicine for heart disease according to [1] where the arrhythmia is a bradyarrhythmia or tachyarrhythmia.
[4] The medicine for heart disease according to [3] where the arrhythmia is any one of extrasystole, ventricular tachycardia, paroxysmal supraventricular tachycardia, atrial flutter, atrial fibrillation, ventricular fibrillation, atrial tachycardia, bundle branch block, Torsade de Pointes, or atrioventricular block.
[5] The medicine for heart disease according to [2] where the disease causing an arrhythmia is any one of myocardial infarction, angina pectoris, heart failure, cardiomyopathy, arrhythmogenic right ventricular cardiomyopathy, sarcoidosis, valvular disease, congenital heart disease, Brugada syndrome, long QT syndrome, or broken heart syndrome.
[6] The medicine for heart disease according to [2] where the disease caused by an arrhythmia is any one of palpitation, dizziness, heart failure, myocardial lesion, cardiogenic brain embolism, cardiac syncope, or cardiogenic shock caused by an arrhythmia.
[7] The medicine for heart disease according to any one of [1] to [6] where the medicine for heart disease is a food product for improving heart disease.
[8] The medicine for heart disease according to any one of [1] to [6] where the medicine for heart disease is a medicinal product for improving heart disease.
[9] The medicine for heart disease according to any one of [1] to [8] where a daily amount per adult of the nicotinamide mononucleotide to be applied is 1 mg to 500 mg.
[10] A method for improving heart disease (excluding medical practice to human), comprising
   causing a target to apply an effective dose of nicotinamide mononucleotide, the target in need thereof.

### Advantageous Effects of Invention

The medicine for heart disease according to the present invention is effective for preventing or treating heart diseases related to arrhythmias. In addition, since nicotinamide mononucleotide, which is an intermediate metabolite related to in vivo NAD⁺ biosynthesis, is contained as an active ingredient, the medicine for heart disease according to the present invention is safe and can be used over a long period of time.

### Brief Description of Drawings

Fig. 1 is an explanatory diagram illustrating metabolic pathways related to niacin (general term for nicotinamide and nicotinic acid).
Fig. 2 is an explanatory diagram indicating a result of an electrocardiogram in Example.
Fig. 3 is an explanatory diagram indicating a result of an electrocardiogram in Example.

### Description of Embodiments

The medicine for heart disease according to the present invention contains nicotinamide mononucleotide (including a salt thereof) as an active ingredient and provides an improving effect on heart diseases. In the present invention, the improvement of heart diseases includes not only the improvement of heart diseases in a narrow sense, but also the alleviation of various symptoms developed from he art diseases, the prevention of heart diseases, and stopping and delaying progress. The detailed reason for obtaining such an action effect by containing nicotinamide mononucleotide as an active ingredient is currently under consideration. However, it is considered that the nicotinamide mononucleotide accelerates"sirtuins" represented by NAD⁺-dependent deacetylases Sirt1 and Sirt3 and consequently eliminates the cause of disorders in the stimulus conduction system, thereby providing an improving effect on heart diseases . Thus, the medicine for heart disease according to the present invention that provides an improving effect on heart diseases can be used as a medicinal product (including a quasi-drug) and a food product such as a functional food. The following describes the present invention in detail.

The nicotinamide mononucleotide (chemical formula: C₁₁H₁₅N₂O₈P) is a compound produced in bodies of many organisms including human, and expressed with a structural formula [Chem. 1] below. The nicotinamide mononucleotide is generally referred to as NMN, and known as an intermediate metabolite involved in a biosynthesis of coenzyme NAD⁺.

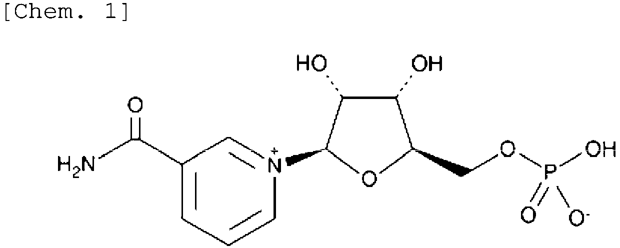

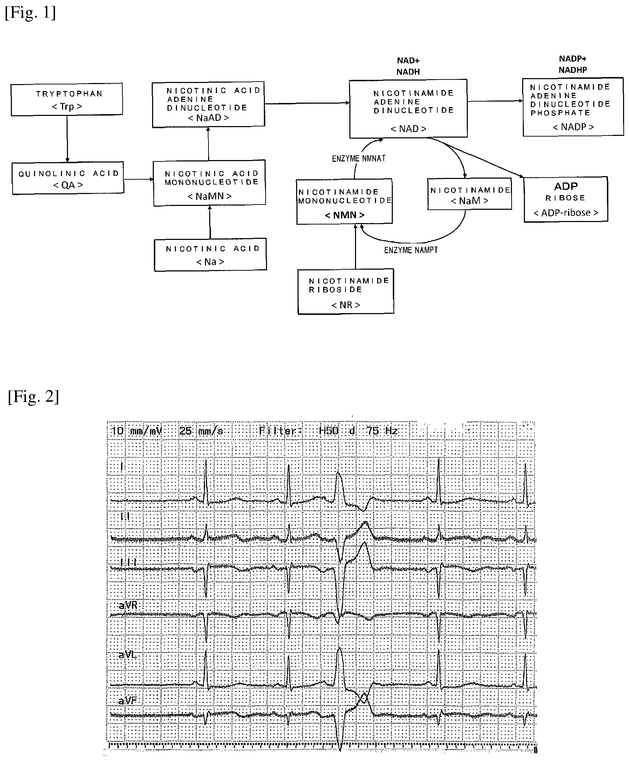

The nicotinamide mononucleotide, which is contained as an active ingredient, is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to Fig. 1. Fig. 1 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

As illustrated in Fig. 1, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN). Meanwhile, when the nicotinic acid (Na) is the starting material, the nicotinic acid is directly converted into the NaMN. Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD. Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730) . Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available from Oriental Yeast Co., ltd. and Bontac Bio-engineering (Shenzhen) Co., Ltd., and those commercial products can be purchased for use.

The medicine for heart disease according to the present invention can be easily manufactured by using nicotinamide mononucleotide alone or by mixing the nicotinamide mononucleotide with other components. The other components are not specifically limited as long as the medicine for heart disease provides the effect of the present invention.

Examples of other components include Na channel blockers (classes Ia group, Ib group, and Ic group), β-blockers (class II group), αβ-blockers, potassium channel blockers (class III group), Ca antagonists (class IV group), digitalis preparations, and the like, which are generally used as anti-arrhythmic drugs, and specifically include medicinal components having an anti-arrhythmic action, such as lidocaine, mexiletine, procainamide, disopyramide, quinidine, propafenone, aprindine, cibenzoline, pirmenol, landiolol, esmolol, flecainide, pilsicainide, bepridil, verapamil, diltiazem, sotalol, amiodarone, nifekalant, nadolol, propranolol, atropine, ATP, and digoxin. In addition, for example, various vitamins, trace amounts of elements, citric acid, malic acid, flavoring agents, inorganic salt, and the like, which are common supplemental components in the food product field may be included as other components.

While the medicine for heart disease according to the present invention can be used for the purpose of improving various heart diseases, it is used mainly for the purpose of improving pathological conditions presenting with an arrhythmia, diseases causing an arrhythmia, or diseases caused by an arrhythmia.

Arrhythmias to become targets in the present invention include both arrhythmias that require medical treatment and arrhythmias that do not require medical treatment. In addition, the arrhythmias include both bradyarrhythmias (heart rate less than or equal to 50 times/minute) and tachyarrhythmias (heart rate greater than or equal to 100 times/minute).

The conditions presenting with an arrhythmia widely mean conditions having an arrhythmia, regardless of the presence or absence of pre-existing conditions, complications, or the like related to the arrhythmia. Accordingly, conditions that cause an arrhythmia without relation to illness, such as aging, physical constitution, fatigue, stress, and lack of sleep, are included. Examples of the conditions presenting with an arrhythmia include extrasystole, ventricular tachycardia, paroxysmal supraventricular tachycardia, atrial flutter, atrial fibrillation, ventricular fibrillation, atrial tachycardia, bundle branch block, Torsade de Pointes, and atrioventricular block.

The diseases causing an arrhythmia specifically include myocardial infarction, angina pectoris, heart failure, cardiomyopathy (dilated, hypertrophic), arrhythmogenic right ventricular cardiomyopathy, cardiac sarcoidosis, valvular disease, congenital heart disease, Brugada syndrome, long QT syndrome, broken heart syndrome, or the like.

The diseases caused by an arrhythmia include palpitation caused by an arrhythmia, dizziness caused by an arrhythmia, sudden death caused by an arrhythmia, heart failure caused by an arrhythmia, myocardial lesion caused by an arrhythmia, cardiogenic brain embolism caused by an arrhythmia, cardiac syncope caused by an arrhythmia, cardiogenic shock caused by an arrhythmia, or the like.

A method for manufacturing the medicine for heart disease is not specifically limited, and a common manufacturing method applied for manufacturing the medicine for heart disease according to its form may be selected as appropriate. For example, when the form is a powder, the medicine for heart disease can be manufactured by appropriately combining nicotinamide mononucleotide, other medicinal components, and components, such as an extending agent, a sweetening agent, a preservation agent, a pH adjuster, and a flavoring agent, and uniformly dispersing and mixing them using equipment having sufficient shearing force and mixing force for manufacturing. Note that, nicotinamide mononucleotide as an active ingredient is distributed in the market and is commercially available. Especially, in recent years, a quality management system and a mass production system of nicotinamide mononucleotide have been established, allowing the nicotinamide mononucleotide to be supplied as a food composition material, and further, the stability of nicotinamide mononucleotide as a food composition has been confirmed.

In the pharmaceutical field, the medicine for heart disease according to the present invention can be used as a medicinal product (including a quasi-drug) for improving heart disease and applied orally or not orally. When it is applied orally, a dosage form of the medicine for heart disease is not specifically limited, but can include, for example, a powder, a tablet, a persistent tablet, a chewable tablet, an effervescent tablet, a troche, a buccal tablet, a sublingual tablet, a capsule formulation, a fine granule, a granule, a pill, a dry syrup, a liquid medicine, a suspending agent, a syrup, and an elixir. Among these forms, considering the ease of taking, the stability of the active ingredient, and the like, the formulation for oral administration such as the powder, the tablet, and the capsule formulation is preferable.

On the other hand, when the medicine for heart disease according to the present invention is applied not orally, examples of the dosage form of the medicine for heart disease include an external preparation, an injection preparation, and a transfusion. However, the external preparation is preferable because the active ingredient can be applied directly into the oral cavity. Specifically, the external preparation can be an ointment, a cream, a toothpaste, a mouthwash, a mouth spray, or the like.

The medicinal product can appropriately contain a known additive for formulation, which is adequate for the dosage form and pharmacologically allowed, considering physicochemical property, biological property, and similar property. Such an additive for formulation is exemplified by, for example, an excipient (lactose, starch, crystalline cellulose, sodium phosphate, and the like), a solvent (water, soybean oil, saline solution, a nonaqueous solvent for injection, and the like), a binder (starch, gelatin, gum arabic, sodium alginate, carmellose sodium, methylcellulose, ethyl cellulose, hydroxypropyl methylcellulose, hydroxypropyl cellulose, polyvinylpyrrolidone, and the like), a disintegrant (starch, carmellose sodium, and the like), a lubricant (talc, magnesium stearate, calcium stearate, macrogol, sucrose fatty acid ester, and the like), a coating agent (white sugar, HPC, shellac, gelatin, glycerin, hydroxypropyl methylcellulose, hydroxypropyl methylcellulose phthalate, cellulose acetate phthalate, and the like), a stabilizer (sodium bisulfite, sodium thiosulfate, sodium edetate, sodium citrate, ascorbic acid, dibutylhydroxytoluene, and the like), a preservative (methyl parahydroxybenzoate, ethyl parahydroxybenzoate, propyl parahydroxybenzoate, benzyl alcohol, phenol, chlorobutanol, benzalkonium chloride, benzethonium chloride, sodium dehydroacetate, thimerosal, and the like), a viscous agent (methylcellulose, carmellose sodium, chondroitin sulfate, sodium alginate, and the like), a suspending agent (various nonionic surfactant, methylcellulose, carmellose sodium, and the like), an emulsifier (gum arabic, cholesterol, sorbitan sesquioleate, polysorbate 80, sodium lauryl sulfate, and the like), a buffer (citric acid, acetic acid, sodium phosphate, and boric acid), a surfactant (hydrogenated castor oil, polysorbate 80, and the like), a colorant (water-soluble food pigment, lake pigment, and the like), a corrigent (lactose, white sugar, glucose, mannitol, and the like), a scenting agent (aromatic essential oils), a plasticizer (the phthalic acid esters, vegetable oils, polyethylene glycol, and the like).

A dose of the medicinal product cannot be equally specified because it differs depending on, for example, age, sex, and weight of a target that takes the food product, the expected effect, and the symptom. However, as the dose of the medicinal product, the daily amount per adult of the nicotinamide mononucleotide to be administered is ordinarily 1 mg to 500 mg, preferably 5 mg to 250 mg, and more preferably 50 mg to 200 mg. Less than 1 mg possibly fails to provide the effect of the present invention, while more than 500 mg merely provides almost similar effect but causes economic disadvantage. The compounding ratio of the nicotinamide mononucleotide in the medicinal product can be appropriately set in accordance with the dosage form, the number of times of the medicinal product administration, and the like so that the daily amount per adult of the nicotinamide mononucleotide to be administered is within the above range.

The number of times of the administration of the medicinal product can be appropriately set in accordance with, for example, the age, weight, and symptom of the administration target and the dose per administration of the medicinal product. The number of times of the medicinal product administration per day can be exemplified by once to three times.

The medicine for heart disease according to the present invention can be used as a food product. In a case of being used as a food product, the medicine for heart disease can be provided as a food product for improving heart disease in the food product field. By daily ingesting the medicine in the form of a food product, the improving effect on heart diseases can be provided repeatedly and is thus especially effectual for enjoying the effect. The type of the food product as the target of the present invention is not specifically limited, and the target includes a functional food, a food for specified health use, a dietary supplement, a supplement, a food additive, a feed, a care food, a diet therapy food, a therapeutic diet, a diet food, and similar food product in addition to general food products. The form of the food product is not limited, and especially in the case of the functional food, the food for specified health use, and the like, the food product can be processed to be provided in the form of, for example, a powder, a tablet, a pill, a granule, a hard capsule formulation, a soft capsule formulation, a jelly, a liquid medicine, a paste medicine, and a chewing gum.

The intake of the food product is different depending on the type of the food product, age, sex, and weight of a target that takes the food product, the expected effect, and the symptom. However, the daily intake per adult of the nicotinamide mononucleotide contained in the food product is ordinarily 1 mg to 500 mg, preferably 5 mg to 250 mg, and more preferably 50 mg to 200 mg. Less than 1 mg possibly fails to provide the effect of the present invention, while more than 500 mg merely provides almost similar effect but causes economic disadvantage. The compounding ratio of the nicotinamide mononucleotide in the food product can be appropriately set in a range of 100% or less so that the daily amount per adult of the nicotinamide mononucleotide to be administered is within the above range.

The food product is safe, and side effects are not specifically recognized. Accordingly, the food product can be ingested over a long period of time not only for the purpose of treating heart diseases but also for the purpose of preventing heart diseases. Therefore, the food product can be applied not only to targets for whom it is desired to treat heart diseases but also to healthy targets so as not to lead to heart diseases.

Since nicotinamide mononucleotide, as described above, has a heart disease improving effect, the present invention further provides a method for improving heart disease that includes causing a target in need thereof. That is, it is a method to improve the heart disease of the target of the ingestion by causing the target to ingest the medicine for heart disease according to the present invention. The target of the ingestion is preferably a mammal such as a human, a mouse, a rat, a rabbit, a dog, a cat, cattle, a horse, a pig, and a monkey, and especially a human is preferable. In the method, the intake of the nicotinamide mononucleotide, the number of intake per day, and similar matters are as described for the medicine for heart disease. The medicine for heart disease can be ingested anytime in any case, and can be ingested by the target over a long period of time.

### Examples

### (Example)

On June 4, 2020, electrocardiography was performed on a subject (64 years old, male) by placing the subject in the supine position, putting clips on the extremities, attaching electrodes to the chest, and using an electrocardiograph (product name: Electrocardiograph ECG-2250, manufactured by NIHON KOHDEN CORPORATION) with leads I to III and leads aVR, aVL, and aVF. The paper feed speed of the electrocardiogram was set to 25 mm/second, and the vertical axis was set to 1 mV = 10 mm for recording. The result is indicated in the electrocardiogram of Fig. 2 (horizontal axis: time (second), vertical axis: electric potential (mV)). On the recorded electrocardiogram, P waves, which appeared earlier than a normal pulse, existed, and QRS complexes, which had a wide and odd-shaped waveform, were recognized. Accordingly, the finding of the recorded electrocardiogram suggested a ventricular extrasystole.

Afterwards, from October 18, 2020, the subject was caused to ingest (take orally) one capsule per day of NMN pure 3000 plus (NMN 50 mg/capsule, manufactured by MIRAILAB BIOSCIENCE Inc., trade name) for 47 consecutive days.

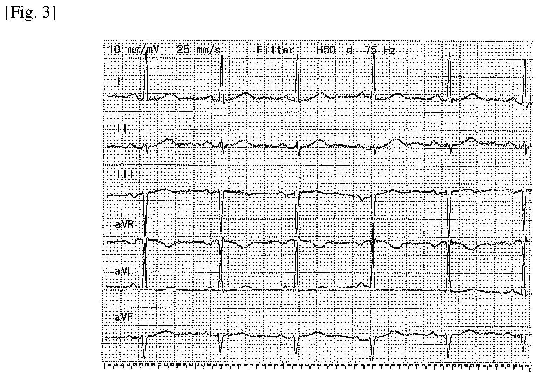

Then, on January 4, 2021, electrocardiography was performed again on the above-described subject similarly as described above . The result is indicated in the electrocardiogram of Fig. 3 (horizontal axis: time (second), vertical axis: electric potential (mV)). As a result, as seen in Fig. 2, the waveforms appeared with regularity in order, the previously recognized QRS complexes having a wide and odd-shaped waveform were not recognized, and the heart rate lowered from before (69 bpm to 57 bpm) . From this, it was confirmed that NMN ingestion was effective for electrocardiogram abnormalities and arrhythmias.

## Claims

1. A medicine for heart disease that contains nicotinamide mononucleotide as an active ingredient.

2. The medicine for heart disease according to claim 1, wherein
the heart disease is a condition presenting with an arrhythmia, a disease causing an arrhythmia, or a disease caused by an arrhythmia.

3. The medicine for heart disease according to claim 1, wherein
the arrhythmia is a bradyarrhythmia or tachyarrhythmia.

4. The medicine for heart disease according to claim 3, wherein
the arrhythmia is any one of extrasystole, ventricular tachycardia, paroxysmal supraventricular tachycardia, atrial flutter, atrial fibrillation, ventricular fibrillation, atrial tachycardia, bundle branch block, Torsade de Pointes, or atrioventricular block.

5. The medicine for heart disease according to claim 2, wherein
the disease causing an arrhythmia is any one of myocardial infarction, angina pectoris, heart failure, cardiomyopathy, arrhythmogenic right ventricular cardiomyopathy, sarcoidosis, valvular disease, congenital heart disease, Brugada syndrome, long QT syndrome, or broken heart syndrome.

6. The medicine for heart disease according to claim 2, wherein
the disease caused by an arrhythmia is any one of palpitation, dizziness, heart failure, myocardial lesion, cardiogenic brain embolism, cardiac syncope, or cardiogenic shock caused by an arrhythmia.

7. The medicine for heart disease according to any one of claims 1 to 6, wherein
the medicine for heart disease is a food product for improving heart disease.

8. The medicine for heart disease according to any one of claims 1 to 6, wherein
the medicine for heart disease is a medicinal product for improving heart disease.

9. The medicine for heart disease according to any one of claims 1 to 8, wherein
a daily amount per adult of the nicotinamide mononucleotide to be applied is 1 mg to 500 mg.

10. A method for improving heart disease (excluding medical practice to human), comprising
causing a target to apply an effective dose of nicotinamide mononucleotide, the target in need thereof.
